(19) **Europäisches Patentamt**

**European Patent Office**

**Office européen des brevets**

(11) **EP 0 794 151 B1**

(12) # EUROPEAN PATENT SPECIFICATION

(45) Date of publication and mention
of the grant of the patent:
**30.05.2001 Bulletin 2001/22**

(51) Int Cl.$^7$: **C01B 25/45**

(21) Application number: **97103907.8**

(22) Date of filing: **08.03.1997**

(54) **Novel metal ion-exchanged phosphorusvanadium compound and solid acid catalyst using the compound**

Durch Metall-Ionen-Austausch gewonnene Phosphor-Vanadiumverbindung und Feste-Säure-Katalysator, der die Verbindung verwendet

Composé de phosphore et de vanadium contenant un ion métallique déposé par échange d'ions et catalyseur acide solide utilisant ce composé

(84) Designated Contracting States:
**BE DE FR GB IT**

(30) Priority: **08.03.1996 JP 5200196**
**08.03.1996 JP 5200296**

(43) Date of publication of application:
**10.09.1997 Bulletin 1997/37**

(73) Proprietor: **NIPPON SHOKUBAI CO., LTD.**
**Chuo-ku, Osaka-shi, Osaka-fu 541 (JP)**

(72) Inventor: **Matsuura, Ikuya**
**Toyama-shi, Toyama-ken (JP)**

(74) Representative:
**Luderschmidt, Schüler & Partner GbR**
**Patentanwälte,**
**John-F.-Kennedy-Strasse 4**
**65189 Wiesbaden (DE)**

(56) References cited:
**EP-A- 0 092 619**    **US-A- 5 288 880**

• **PATENT ABSTRACTS OF JAPAN vol. 006, no. 202 (C-129), 13 October 1982 & JP 57 111219 A (MITSUBISHI KASEI KOGYO KK), 10 July 1982,**
• **PATENT ABSTRACTS OF JAPAN vol. 007, no. 022 (C-148), 28 January 1983 & JP 57 177347 A (NIPPON SHOKUBAI KAGAKU KOGYO KK), 1 November 1982,**
• **PATENT ABSTRACTS OF JAPAN vol. 007, no. 056 (C-155), 8 March 1983 & JP 57 207545 A (MITSUBISHI KASEI KOGYO KK), 20 December 1982,**

Remarks:
The file contains technical information submitted after the application was filed and not included in this specification

**Description**

Field of the Invention:

This invention relates to a novel metal ion-exchanged phosphorus-vanadium oxide and to a solid acid catalyst which contains this phosphorus-vanadium oxide. More particularly, this novel phosphorus-vanadium oxide is a compound which exhibits a specific d value (lattice spacing, Å). This compound manifests the qualities of a solid acid and, owing to the acid qualities, can be used as a catalyst.

Description of the Prior Art:

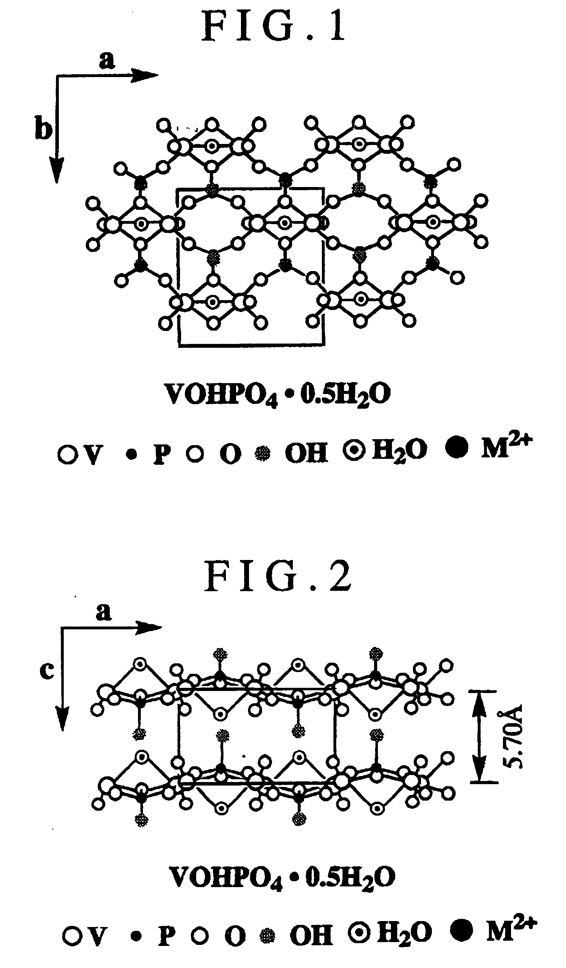

Various studies have been heretofore made concerning the phosphorus-vanadium oxides and physical properties and uses of these oxides have been simultaneously explored for the sake of development. The phosphorus-vanadium compounds are specific in such qualities as the state of oxidation and the configuration and consequently have various crystal structures and find various applications owing to these structures. It is well known that the catalytically effective component of phosphorus-vanadium oxides is divanadyl pyrophosphate, a crystalline oxide which has the composition of $(VO)_2P_2O_7$. This divanadyl pyrophosphate is obtained by synthesizing its precursor, vanadyl hydrogen orthophosphate hydrate ($VOHPO_4 \cdot nH_2O$), and thermally dehydrating this precursor. It is also known that the vanadyl hydrogen orthophosphate hydrate as the precursor is an interlayer compound which is formed by the hydrogen bondage of opposed $VOHPO_4$ layers through the medium of water molecules intervening therebetween.
It is widely known that phosphorus-vanadium compounds generally form an effective catalyst for gas-phase oxidation of such hydrocarbons as butane, butene, and butadiene which have four carbon atoms (hereinafter referred to briefly as "C4 hydrocarbons") . Various patterns of X-ray diffraction peaks for identifying these phosphorus-vanadium oxides have been published (JP-A-53-61,588, JP-A-56-41,816, JP-A-56-45, 815, JP-A-59-132,938, and JP-A-05-15,781). Various inventions concerning the addition of third components to the phosphorus-vanadium oxides have been also disclosed (JP-A-52-135,580, JP-A-54-30,114, and JP-A-57-111,219).
Reports on phosphorus-vanadium oxides are published in various articles of literature besides the patent publications mentioned above. B. K. Hodnett. ed., Catalysts Today, Vol. 1, No. 5 (1987), for example, carries a detail report.
No attempt has ever been made to synthesize a compound having a divalent metal cation exchanged for the $H^+$ ion which intervenes between the opposed layers of the vanadyl hydrogen orthophosphate hydrate, a precursor of a phosphorus-vanadium oxide.
It is well known that the phosphorus-vanadium compound having such a plane structure as illustrated in the form of a model in Fig. 1, e.g. a vanadyl hydrogen orthophosphate hydrate, $VOHPO_4$, is a layer compound which results from the hydrogen bondage through the medium of constitution water intervening between the $VOHPO_4$ layers as shown in Fig. 2. The interlayer distance is generally 5.70 Å. Absolutely no case of having this interlayer proton exchanged with other metal cation has been known to date.
An object of this invention, therefore, is to provide a novel phosphorus-vanadium compound.
Another object of this invention is to provide a novel phosphorus-vanadium compound which is obtained by the exchange of a proton present between opposed layers in the layer structure of a phosphorus-vanadium compound with a metal ion and a method for the production thereof.
Yet another object of this invention is to provide a novel solid acid catalyst.
A further object of this invention is to provide a catalyst appropriate for gas-phase partial oxidation of a hydrocarbon.

SUMMARY OF THE INVENTION

The objects mentioned above are accomplished by the following items, (1) - (8).

(1) A metal ion-exchanged phosphorus-vanadium compound obtained by treating vanadyl hydrogen orthophosphate hydrate represented by the formula (1)

$$VOHPO_4 \cdot nH_2O \qquad (1)$$

wherein n fulfills the expression, $0 \leqq n \leqq 2.0$, with an aqueous divalent metal salt solution thereby effecting the exchange of $H^+$ present between the layers of the vanadyl hydrogen orthophosphate hydrate and drying the resultant ion exchanger.

(2) A compound according to Item (1) above, wherein the interlayer distance is in the range of 7.0 to 8.2 Å.

(3) A metal ion-exchanged phosphorus-vanadium compound obtained by treating vanadyl hydrogen orthophosphate hydrate represented by the formula (1)

$$VOHPO_4 \cdot nH_2O \qquad\qquad (1)$$

wherein n fulfills the expression, $0 \leqq n \leqq 2.0$, with an aqueous divalent metal salt solution thereby effecting the exchange of $H^+$ present between the layers of the vanadyl hydrogen orthophosphate hydrate, drying the resultant ion exchanger, and calcining the dried ion exchanger.

(4) A compound according to Item (1) or Item (3) above, wherein the divalent metal is at least one member selected from the group consisting of cobalt, nickel, copper, zinc, manganese, iron, magnesium, palladium, platinum, and germanium.

(5) A method according to Item (1) or Item (3) above, wherein the drying is carried out at a temperature in the range of 150° to 250 °C.

(6) A method according to Item (3), wherein the calcination is carried out in the presence of a hydrocarbon under an oxidizing atmosphere at a temperature in the range of 350° to 600 °C.

(7) A solid catalyst formed of a metal ion-exchanged phosphorus-vanadium compound set forth in any of Items (1) - (6).

(8) A catalyst for gas-phase partial oxidation of a hydrocarbon, formed of a metal ion-exchanged phosphorus-vanadium compound set forth in any of Items (1) - (6).

BRIEF DESCRIPTION OF THE DRAWINGS

Fig. 1 is a model plan view illustrating the structure of vanadyl hydrogen orthophosphate hydrate.

Fig. 2 is a model diagram illustrating a cross section of the structural diagram shown in Fig. 1.

Fig. 3 is a model diagram illustrating the structure of vanadyl hydrogen orthophosphate hydrate and the cross-sectional structure of an ion exchanger thereof.

Fig. 4 is an X-ray diffraction chart illustrating one example of a metal ion-exchanged phosphorus-vanadium compound according to this invention.

Fig. 5 is an X-ray diffraction chart illustrating another example of the metal ion-exchanged phosphorus-vanadium compound according to this invention.

DESCRIPTION OF THE PREFERRED EMBODIMENT

The metal ion-exchanged phosphorus-vanadium compound in accordance with this invention is obtained by causing a vanadium compound to react with a phosphorus compound thereby forming vanadyl hydrogen orthophosphate hydrate represented by the formula (1)

$$VOHPO_4 \cdot nH_2O \qquad\qquad (1)$$

wherein n fulfills the expression, $0 \leqq n \leqq 2.0$, treating the vanadyl hydrogen orthophosphate hydrate with an aqueous divalent metal salt solution thereby effecting the exchange of $H^+$ present between the layers of the vanadyl hydrogen orthophosphate hydrate and drying the resultant ion exchanger. The dried ion exchanger, when necessary, is further calcined.

The interlayer distance of the metal ion-exchanged phosphorous-vanadium compound in accordance with the present invention is 7.0- 8.2 Å, preferably 7.1- 8.1 Å

The method for the production of the metal ion-exchanged phosphorus-vanadium compound according to this invention will be described in detail below.

For the purpose of this production, this invention permits adoption of any method which is capable of obtaining a product exhibiting a specific X-ray diffraction spectrum which will be described afterward. The typical methods available for the production are known in the following type kinds, depending on the water content of the vanadyl hydrogen orthophosphate hydrate ($VOHPO_4 \cdot nH_2O$). First, the raw materials to be used for the preparation of the catalyst will be explained.

The vanadium source may be any of the vanadium compounds which are generally adopted for the production of phosphorus-vanadium oxides. As typical examples of the vanadium compounds, pentavalent, tetravalent, or trivalent vanadium-containing compounds such as vanadium pentoxide, metavanadates, and vanadium oxyhalogenides may be cited. Among other vanadium compounds mentioned above, vanadium pentoxide proves particularly advantageous.

The phosphorus source, similarly to the vanadium source, may be any of the phosphorus compounds which are generally adopted for the production of phosphorus-vanadium oxides. As typical examples of the phosphorus compounds, orthophosphoric acid, pyrophosphoric acid, phosphorous acid, polyphosphoric acid, and phosphorus pentoxide may be cited. Among other phosphorus compounds mentioned above, orthophosphoric acid proves particularly advantageous.

Method 1 for preparation

Method 1 for preparation will be explained. The content of $H_2O$ in a compound is represented by a specific amount for the sake of convenience.

The method 1 for preparation comprises causing a vanadium compound to react with a phosphorus compound in an aqueous organic solvent thereby forming vanadyl hydrogen orthophosphate hydrate such as, for example, $VOHPO_4 \cdot 0.5H_2O$, combining the vanadyl hydrogen orthophosphate hydrate with distilled water and further with an alkali salt, then adjusting the pH value of the resultant mixture to 5 - 7, filtering and cleaning the mixture, and treating the cleaned mixture with an aqueous metal salt solution thereby effecting required ion exchange. Then, the resultant ion exchanger is dried and the dried ion exchanger such as, for example, $VOM_{0.5}PO_4$ is calcined when necessary. More specifically, the preparation is carried out by the following procedure.

(1) step of reaction of vanadium compound with phosphorus compound

This step obtains vanadyl hydrogen orthophosphate hydrate such as, for example, $VOHPO_4 \cdot 0.5H_2O$ by the reaction of a vanadium compound with a phosphorus compound in an aqueous solvent or organic solvent.

The aqueous solvent or organic solvent for the reaction of the vanadium compound with the phosphorus compound is required to combine the functions of a reducing agent for reducing the pentavalent vanadium compound and of a reaction solvent. Any of the aqueous organic solvents combining these functions can be used. As typical examples of these organic solvents, aliphatic alcohols such as isopropanol, 2-butanol, isobutanol, 2-pentanol, and isopentanol; aromatic alcohols such as benzyl alcohol, methylbenzyl alcohol, dimethylbenzyl alcohol, and ethylbenzyl alcohol; and aromatic aldehydes such as benzaldehyde, tolualdehyde, dimethyl benzaldehyde, and anisaldehyde may be cited. As the organic solvent, aqueous organic solvent is preferable. It is allowable to use aliphatic alcohols, aromatic alcohols, and aromatic aldehydes as suitably mixed. Preferably, isobutanol and benzyl alcohol are used either singly or together in a mixed state. Further, when the reaction of the vanadium compound with the phosphorous compound is carried out in the aqueous solvent, concentrated hydrochloric acid, hydrazine or hydroxylamine may be used as a reducing agent which can reduce the pentavalent vanadium to tetravalent vanadium compound.

The ratio of the amounts of the phosphorus source and the vanadium source to be used is properly in the range of 0.9/1 - 1.2/1, preferably 0.95/1 - 1.1/1 as phosphorus/vanadium (atomic ratio).

The reaction of the vanadium compound with the phosphorus compound carried out in the solvent mentioned above at a temperature in the range of 60° - 150°C, preferably 80° - 140 °C, for a period in the range of 2 - 24 hours, preferably 4 - 12 hours produces the vanadyl hydrogen orthophosphate hydrate.

(2) Step for pH adjustment

This step comprises combining the produced vanadyl hydrogen orthophosphate hydrate with distilled water and further with an alkali salt, then adjusting the pH value of the resultant mixture to 5 - 7, preferably 6 - 7 , and filtering and cleaning the mixture.

The pH adjustment is effected generally with an alkali solution. The vanadyl hydrogen orthophosphate hydrate and distilled water added thereto are together stirred thoroughly. The resultant mixture, by gradual addition of the aqueous solution of such alkali as sodium hydroxide or potassium hydroxide, has the pH value thereof adjusted to 5 - 7, preferably 6 - 7 . After the pH adjustment, the precipitate is immediately separated by filtration and thoroughly cleaned.

(3) Step for metal ion exchange

This step comprises combining the washed precipitate with distilled water and subjecting the resultant mixture to a treatment with an aqueous metal salt solution to effect the ion exchange.

The resultant precipitate is added to the aqueous solution of a metal salt and then heated at a temperature in the range of 60° - 100°C, preferably 80° - 100 °C, for a period in the range of 5 -60 hours, preferably 15 - 60 hours, to induce an ion- exchange reaction as required. The metal salts available for this treatment include nitrates, carbonates, and organic acid salts. Preferably, divalent metal salts of such organic acids as acetic acid are used. The metal is appropriately used in an amount in the range of 0.5 - 5 mols per mol of the $VOHPO_4 \cdot 0.5H_2O$. As typical examples of the divalent metal, cobalt, nickel, copper, zinc, manganese, iron, magnesium, palladium, platinum, and germanium may be cited. Among other divalent metals mentioned above, cobalt, nickel, copper, and zinc prove particularly advantageous. These divalent metals may be used either singly or in the form of a mixture of two or more members. The product of the ion-exchange reaction is filtered and washed to remove the excess metal salt.

(4) Step for drying

This step comprises drying the precipitate consequently obtained. The precipitate is dried in the stream of an inert gas or in the current of air at a temperature in the range of 150° - 250°C, preferably 180° - 220 °C, for a period in the range of 5 - 14 hours, preferably 12 - 20 hours, to obtain an ion exchanger such as, for example $VOM_mPO_4$ (wherein m represents a numerical value in the range of 0.01 - 0.5, preferably 0.1 - 0.5.

(5) Step for calcination

This step comprises calcining the dried precipitate after the step for drying. This step is performed optionally.
The dried ion exchanger such as, for example, $VOM_{0.5}PO_4$, is pulverized or molded and then calcined in the stream of an inert gas or in the stream of a hydrocarbon gas such as butane, preferably in the stream of a mixed gas of hydrocarbon gas with air, at a temperature in the range of 350° - 600°C, preferably 400° - 600 °C, for a period in the approximate range of 5 - 24 hours, preferably 10 - 20 hours. A concentration of hydrocarbon gas is in the range of 0.5 - 10% by volume, preferably 5 - 10% by volume calculated, as butane to total of the hydrocarbon gas and air.
Now, Method 2 for preparation will be explained. The content of the water of crystallization, $H_2O$, in a compound is represented by a specific amount for the sake of convenience. The method 2 for preparation comprises refluxing a vanadium compound and a phosphorus compound in an aqueous solvent thereby forming $VOPO_4 \cdot 2.0H_2O$, reducing the produced $\alpha$-$VOPO_4 \cdot 2.0H_2O$ further in an organic solvent thereby producing a vanadyl hydrogen orthophosphate hydrate such as, for example, $VoHPO_4 \cdot 1.5H_2O$, and treating this compound with an aqueous solution of a metal salt thereby inducing an ion exchange. This preparation is carried out more specifically by the following procedure.

(1) Step for reaction of vanadium compound with phosphorus compound

This step comprises refluxing a vanadium compound and a phosphorus compound in an aqueous solvent thereby forming $VOPO_4 \cdot 2.0H_2O$.
The ratio of the amounts of the phosphorus source and the vanadium source, i.e. phosphorus/vanadium (atomic ratio), is in the range of 5/1 - 12/1, preferably 7/1 - 10/1.
The $VOPO_4 \cdot 2.0H_2O$ can be obtained by adding the vanadium source and the phosphorus source to distilled water and refluxing the resultant mixture at a temperature in the range of 60° - 100°C, preferably 80° - 100 °C, for a period in the range of 2 - 12 hours, preferably 6 - 10 hours.

(2) Step for reducing $VOPO_4 \cdot 2.0H_2O$

This step comprises reducing the $\alpha$-$VOPO_4 \cdot 2.0H_2O$ obtained at the step mentioned above in an organic solvent thereby obtaining $VOHPO_4 \cdot 1.5H_2O$. The solvents usable for reducing $VOPO_4 \cdot 2.0H_2O$ include such 1-alkanols as 1-propanol, 1-butanol, and 1-pentanol. preferably 1-butanol. The $VOHPO_4 \cdot 1.5H_2O$ is obtained by treating $VOPO_4 \cdot 2.0H_2O$ in the solvent mentioned above at a temperature in the range of 60° - 150°C, preferably 80° - 110 °C, for a period in the range of 2 - 24 hours, preferably 8 - 12 hours.

(3) Step for metal ion exchange

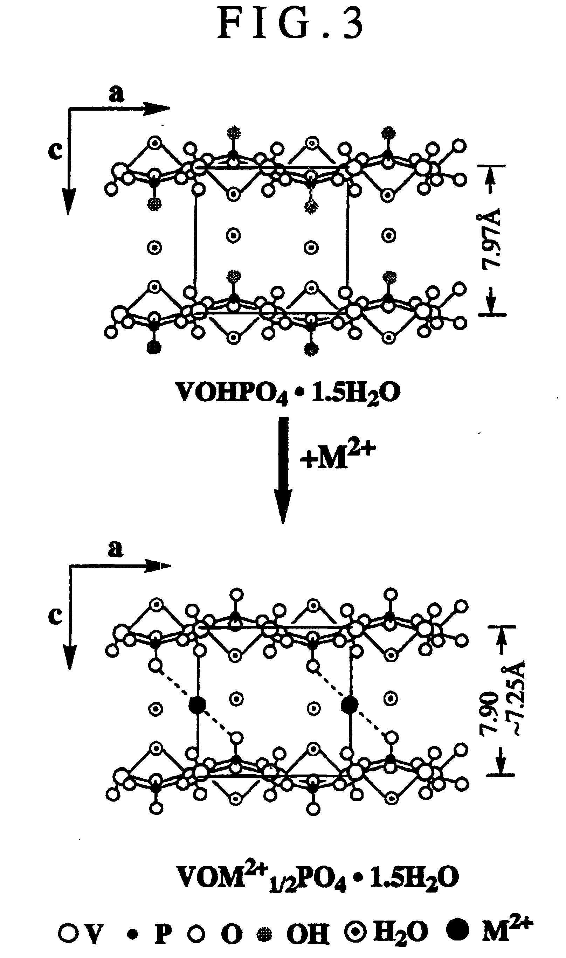

This step comprises treating the $VOHPO_4 \cdot 1.5H_2O$ having such a structure as shown in Fig. 3 with the aqueous solution of a metal salt thereby inducing an ion-exchange reaction. The ion-exchange reaction is carried out by combining the produced $VOHPO_4 \cdot 1.5H_2O$ with the aqueous solution of a metal salt and refluxing the resultant mixture at a temperature in the range of 60° - 100°C, preferably'80° - 100 °C, for a period in the range of 5- 60 hours, preferably 15 - 60 hours. The metal salts available for this treatment include nitrates, sulfates, carbonates, and organic acid salts. Preferably, divalent metal salts of such organic acids as acetic acid are used. Appropriately, the metal is used in an amount in the range of 0.5 - 5 mols, preferably 3 - 5 mols per mol of the $VOHPO_4 \cdot 0.5H_2O$. As typical examples of the divalent metal, cobalt, nickel, copper, zinc, manganese, iron, magnesium, palladium, platinum, and germanium may be cited. Among other divalent metals mentioned above, cobalt, nickel, copper, and zinc prove particularly advantageous. These divalent metals may be used either singly or in the form of a mixture of two or more members. The product of the ion-exchange reaction is filtered and washed to remove the excess metal salt.

(4) Step for drying

This step comprises drying the ion exchanger obtained in the form of a precipitate. The precipitate is dried in the stream of an inert gas such as nitrogen or in a stream of air at a temperature in the range of 150° - 250°C, preferably 180° - 220 °C, for a period in the range of 5 - 24 hours, preferably 12 - 20 hours to obtain $VOM_mPO_4$. In this formula, m is a numerical value in the range of 0.01 - 0.5, preferably 0.1 - 0.5.

(5) Step for calcination

This step comprises calcining the dried precipitate. It is carried out optionally. The dried ion exchanger such as, for example, $VOM_mPO_4$, is pulverized or molded and then calcined in the stream of an inert gas or in the stream of a hydrocarbon gas such as butane, preferably in the stream of a mixed gas of hydrocarbon gas with air, at a temperature in the range of 350° - 600°C, preferably 400 - 500 °C, for a period in the approximate range of 5 - 24 hours, preferably 10 - 20 hours. A concentration of hydrocarbon gas is in the range of 0.5 - 10% by volumes, preferably 5 - 10% by volume calculated as butane to total of the hydrocarbon gas and air.

By the steps mentioned above, the vanadium-phosphorus oxide of this invention is obtained.

When the metal ion-exchanged phosphorus-vanadium compound obtained by the method 1 for preparation or the method 2 for preparation mentioned above is used as a catalyst, the compound can be used as molded in a specific form. The molding can be carried out in the presence of a molding aid. As typical examples of the molding air to be effectively used herein, inorganic substances such as silica gel, alumina sol, and talc and organic substances such as graphite and fatty acid salts may be cited. Further, the molding may be carried out in the presence of fibers of an inorganic substance. The catalyst of this invention for gas-phase oxidation can be used either singly or can be used as molded together with such a carrier as silica, alumina, titania, silicon carbide, or ceramic substance or deposited on such a carrier. It has no particular limit to impose as to shape. It can be molded in the form of powder, spheres, cylinders, arcs, or saddles by such known methods as tableting or extrusion molding.

As typical examples of the catalytic gas-phase oxidation reaction, the reactions for the production of maleic anhydride by the oxidation of butane, the production of methacrolein and methacrylic acid by the oxidation of isobutane, the production of methacrylic acid by the oxidation of methacrolein, the production of acrylonitrile by the ammoxidation of propane, and the production of methacrylic acid by the oxydehydrogenation of isobutyric acid may be cited. The catalyst can be particularly used for the selective oxidation of normal butane into maleic anhydride in the presence of molecular oxygen.

Now, this invention will be described more specifically below with reference to working examples.

Example 1

In 100 ml of distilled water, 6.0 g of $VOHPO_4 \cdot 0.5H_2O$ which was prepared by the reaction of vanadium pentoxide ($V_2O_5$) in isobutanol with 99% orthophosphoric acid at 80°C was stirred. The resultant mixture was adjusted to pH 6.5 by gradual addition of an aqueous 1.0 mol/liter sodium hydroxide solution. The mixture was immediately filtered and repeatedly washed to remove excess sodium ions. This precipitate and 250 ml of an aqueous 0.2 mol/liter divalent cobalt acetate solution were together refluxed at 80°C for 48 hours. The refluxed mixture was filtered and washed to remove excess metal ions. The cleaned mixture was dried overnight at room temperature and further dried in a stream of argon at 200°C for 3 hours. The dried mixture was confirmed to be a divalent cobalt ion exchanger of $VOM_{0.5}PO_4$. The cobalt ion exchanger thus obtained was calcined in the stream of air-mixed gas having a normal butane concentration of 2.0 vol% at 480°C for 12 hours.

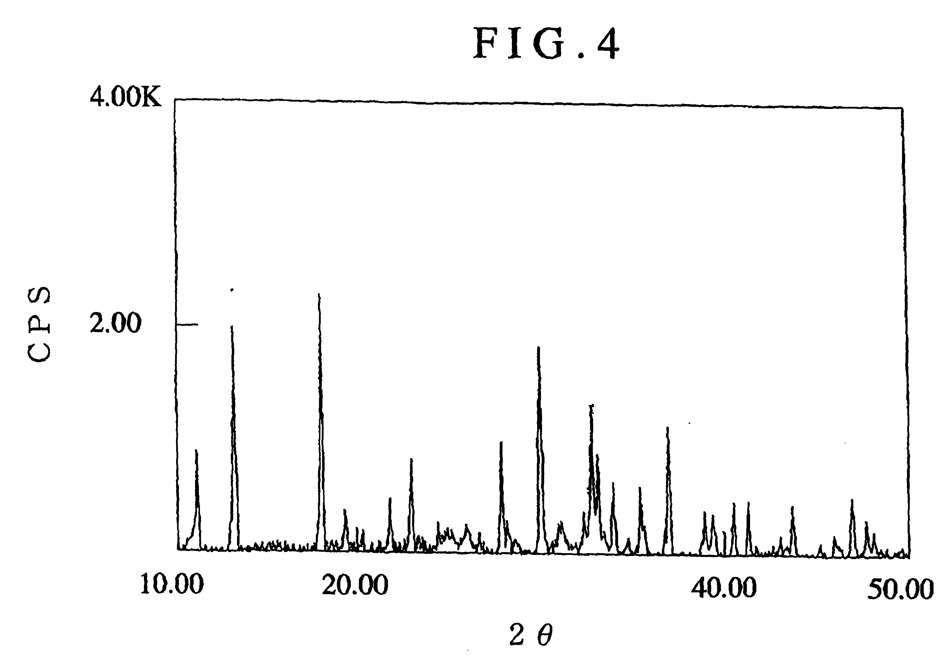

The X-ray diffraction spectrum of a dry mass of the produced cobalt ion-exchanged phosphorus-vanadium oxide ($VOCo_{0.5}PO_4$) was found to have such main peaks as shown in Table 1. The X-ray diffraction spectrum of $VOHPO_4 \cdot 1.5H_2O$ was also shown in Table 1 for reference. Further, lattice constants of $VOHPO_4 \cdot 1.5H_2O$ and the cobalt ion-exchanged phosphorous-vanadium oxide are shown in Table 2. The X-ray diffraction chart of this compound was as shown in Fig. 4.

The X-ray diffraction spectrum of a sintered mass of the cobalt-exchanged phosphorus-vanadium oxide ($VOCo_{0.5}PO_4$) was found to have such main peaks as shown in Table 3.

Example 2

$VOHPO_4 \cdot 1.5H_2O$ was prepared by refluxing vanadium pentoxide ($V_2O_5$) and 85% orthophosphoric acid in distilled water to form $VOPO_4 \cdot 2.0H_2O$ and refluxing the produced compound in 1-butanol at 110°C. In 250 ml of an aqueous 0.2 mol/liter divalent cobalt acetate solution, 6.0 g of the ($VOHPO_4 \cdot 1.5H_2O$) was refluxed at 80°C for 48 hours. The mixture resulting from the reflux was filtered and washed to remove excess metal ions. The cleaned mixture was dried overnight at room temperature. It was further dried in a stream of argon at 200°C for 3 hours. This dried mixture was found to be a divalent cobalt ion exchanger of ($VOM_{0.5}PO_4$). The cobalt ion exchanger was calcined in a stream of air-mixed gas having a normal butane concentration of 2.0 vol% at 480°C for 12 hours.

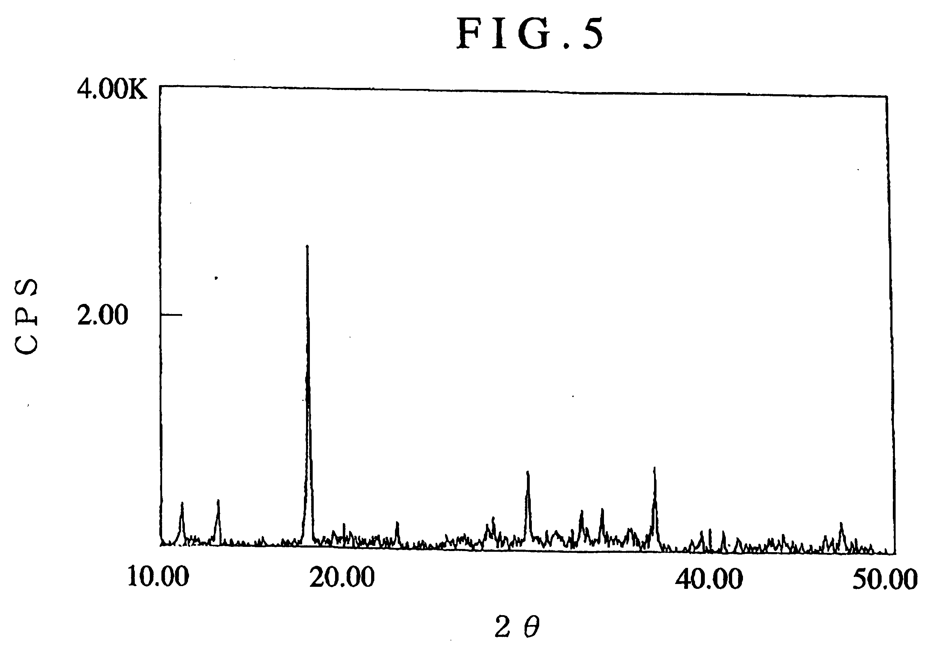

The X-ray diffraction spectrum of a dried mass of the produced cobalt ion-exchanged phosphorus-vanadium oxide ($VOCo_{0.6}PO_4$) was found to have such main peaks as shown in Table 1. The X-ray diffraction chart of this compound was as shown in Fig. 5.

The X-ray diffraction spectrum of a sintered mass of the produced cobalt-exchanged phosphorus-vanadium oxide ($VOCo_{0.6}PO_4$) was found to have such main peaks as shown in Table 3.

Example 3

In 100 ml of distilled water, 6.0 g of $VOHPO_4 \cdot 0.5H_2O$ which was prepared by the reaction of vanadium pentoxide ($V_2O_5$) in 2-butanol with 99% orthophosphoric acid at 80°C was stirred. The resultant mixture was adjusted to pH 6.5 by gradual addition of an aqueous 1.0 mol/liter sodium hydroxide solution. The mixture was immediately filtered and repeatedly washed to remove excess sodium ions. This precipitate and 250 ml of an aqueous 0.2 mol/liter divalent nickel acetate solution were together refluxed at 90°C for 55 hours. The refluxed mixture was filtered and washed to remove excess metal ions. The cleaned mixture was dried overnight at room temperature and further dried in a stream of argon at 200°C for 3 hours. The dried mixture was confirmed to be a divalent nickel ion exchanger of $VOM_{0.5}PO_4$. The nickel ion exchanger thus obtained was calcined in the steam of air-mixed gas having a normal butane concentration of 2.0 vol% at 460°C for 12 hours.

The X-ray diffraction spectrum of a dry mass of the produced nickel ion-exchanged phosphorus-vanadium oxide ($VONi_{0.5}PO_4$) was found to have such main peaks as shown in Table 1. Lattice constant of the nickel ion exchanged phosphorous-vanadium oxide is shown in Table 2.

The X-ray diffraction spectrum of a sintered mass of the nickel-exchanged phosphorus-vanadium oxide ($VOMi_{0.5}PO_4$) was found to have such main peaks as shown in Table 3.

Example 4

$VOHPO_4 \cdot 1.5H_2O$ was prepared by refluxing vanadium pentoxide ($V_2O_5$) and 85% orthophosphoric acid in distilled water to form $VOPO_4 \cdot 2.0H_2O$ and refluxing the produced compound in 1-butanol at 110°C. In 250 ml of an aqueous 0.2 mol/liter divalent nickel acetate solution, 6.0 g of the ($VOHPO_4 \cdot 1.5H_2O$) was refluxed at 85°C for 60 hours. The mixture resulting from the reflux was filtered and washed to remove excess metal ions. The cleaned mixture was dried overnight at room temperature. It was further dried in a stream of argon at 200°C for 3 hours. This dried mixture was found to be a divalent nickel ion exchanger of ($VOM_{0.5}PO_4$). The nickel ion exchanger was calcined in a stream of air-mixed gas having a normal butane concentration of 2.0 vol% at 480°C for 12 hours.

The X-ray diffraction spectrum of a dried mass of the produced nickel-exchanged phosphorus-vanadium oxide ($VOCo_{0.5}PO_4$) was found to have such main peaks as shown in Table 1.

The X-ray diffraction spectrum of a sintered mass of the produced nickel-exchanged phosphorus-vanadium oxide ($VOCo_{0.6}PO_4$) was found to have such main peaks as shown in Table 3.

Example 5

In 100 ml of distilled water, 6.0 g of $VOHPO_4 \cdot 0.5H_2O$ which was prepared by the reaction of vanadium pentoxide ($V_2O_5$) in 2-butanol with 99% orthophosphoric acid at 80°C was stirred. The resultant mixture was adjusted to pH 6.5 by gradual addition of an aqueous 1.0 mol/liter sodium hydroxide solution. The mixture was immediately filtered and repeatedly washed to remove excess sodium ions. This precipitate and 250 ml of an aqueous 0.2 mol/liter divalent copper acetate solution were together refluxed at 90°C for 55 hours. The refluxed mixture was filtered and washed to remove excess metal ions. The cleaned mixture was dried overnight at room temperature and further dried in a stream of argon at 200°C for 3 hours. The dried mixture was confirmed to be a divalent copper ion exchanger of $VOM_{0.5PO}4$. The copper ion exchanger thus obtained was calcined in the steam of air-mixed gas having a normal butane concentration of 2.0 vol% at 480°C for 12 hours.

The X-ray diffraction spectrum of a dry mass of the produced copper ion-exchanged phosphorus-vanadium oxide ($VONi_{0.5}PO_4$) was found to have such main peaks as shown in Table 1. Lattice constant of the copper ion-exchanged phosphorous-vanadium oxide is shown in Table 2.

Example 6

$VOHPO_4 \cdot 1.5H_2O$ was prepared by refluxing vanadium pentoxide ($V_2O_5$) and 85% orthophosphoric acid in distilled water to form $VOPO_4 \cdot 2.0H_2O$ and refluxing the produced compound in 1-butanol at 110°C. In 250 ml of an aqueous 0.2 mol/liter divalent zinc acetate solution, 6.0 g of the ($VOHPO_4 \cdot 1.5H_2O$) was refluxed at 80°C for 30 hours. The mixture resulting from the reflux was filtered and washed to remove excess metal ions. The cleaned mixture was dried overnight at room temperature. It was further dried in a stream of argon at 200°C for 3 hours. This dried mixture was found to be a divalent zinc ion exchanger of ($VOM_{0.4}PO_4$). The zinc ion exchanger was calcined in a stream of air-mixed gas having a normal butane concentration of 2.0 vol% at 480°C for 12 hours.
The X-ray diffraction spectrum of a dried mass of the produced zinc-exchanged phosphorus-vanadium oxide ($VOZn_{0.4}PO_4$) was found to have such main peaks as shown in Table 1. Lattice constant of the zinc ion-exchanged phosphorus oxide is shownlin Table 2.

Table 1

| | VOHPO$_4$·1.5H$_2$O | | Ni | | Co | | Cu | | Zn | |
|---|---|---|---|---|---|---|---|---|---|---|
| hkl | d value (Å) | strength | d value (Å) | strength | d value (Å) | strength | d value (Å) | strength | d value (Å) | strength |
| 001 | 7.97 | S | 7.9 | W | 7.85 | W | 7.31 | VS | 7.25 | VS |
| | | | 6.71 | W | 6.64 | W~M | | | 6.56 | W |
| 020 | 4.81 | W | 4.87 | W | 4.89 | VS | 4.85 | S | 4.6 | W |
| | | | 3.81 | W | 3.85 | W | 3.77 | M | | W |
| 112 | 3.30 | W | 3.3 | W | 3.2 | W | | | | W |
| 220 | 2.94 | VS | 2.94 | W | 2.98 | W | 2.93 | S | 2.98 | W |
| 202 | 2.72 | W | 2.7 | W | 2.71 | W | | | | W |
| | | | 2.61 | W | 2.62 | W | 2.64 | S | 2.63 | W |
| 040 | 2.41 | W | 2.39 | W | 2.42 | M | 2.43 | W | 2.47 | W |

EP 0 794 151 B1

Table 2

| Lattice Const. (Å) | a | b | c |
|---|---|---|---|
| $VOHPO_4 \cdot 1.5H_2O$ | 7.43 | 9.62 | 7.97 |
| $VONi_{0.5}PO_4 \cdot 1.5H_2O$ | 7.43 | 9.74 | 7.90 |
| $VOCo_{0.5}PO_4 \cdot 1.5H_2O$ | 7.43 | 9.78 | 7.84 |
| $VOCu_{0.5}PO_4 \cdot 1.5H_2O$ | 7.43 | 9.70 | 7.31 |
| $VOZn_{0.5}PO_4 \cdot 1.5H_2O$ | 7.43 | 9.88 | 7.25 |

A factor showing the interlayer distance of the phousphorous-vanadium compound is hkl=1 in Table 1 and the value of c axis about the lattice constant.

Table 3

| Ni | | Co | |
|---|---|---|---|
| d value (Å) | Strength | d value (Å) | Strength |
| 5.86 | W | 5.79 | W |
| 4.46 | W | 4.43 | W~M |
| 3.45 | W | 3.4 | W~M |
| 3.07 | S | 3.04 | VS |
| 2.95 | W | 2.89 | W~M |
| 2.57 | VS | 2.55 | W~M |
| 2.14 | w | 2.12 | w |

Examples 7 - 10

Synthesis of maleic anhydride by oxidation of n-butane

The terms "conversion," "selectivity," and "yield" are defined as follows.

Conversion (mol%) = (Number of mols of butane consumed by reaction/number of mols of butane fed to the reaction) x 100

Selectivity (mol%) = (Number of mols of formed maleic anhydride/number of mols of butane consumed by reaction) x 100

Yield (mol%) = (Conversion Selectivity) x 100

A sample, 10 g in weight, of each metal ion-exchanged vanadium-phosphorus oxides obtained in Example 1 and Examples 4 - 6 was molded in the form of pellets, 5 mm in diameter and 5 mm in height. A flow type reaction vessel was packed with the pellets. A mixed gas comprising n-butane with air having a n-butane concentration of 1.5 vol% was introduced at a spatial velocity of 2000 hr$^{-1}$ into the reaction vessel to induce gaseous-phase oxidation of n-butane. The results are shown in Table 4.

Table 4

| Example | Catalyst | Reaction temperature (°C) | Conversion (%) | Selectivity(%) |
|---|---|---|---|---|
| Example 7 | Catalyst of Ex.1 (Co) | 450 | 75 | 58 |
| Example 8 | Catalyst of Ex.4 (Ni) | 450 | 80 | 52 |
| Example 9 | Catalyst of Ex.5 (Cu) | 450 | 85 | 49 |
| Example 10 | Catalyst of Ex.6 (Zn) | 450 | 80 | 57 |

Examples 11 - 14

Synthesis of methacrolein and nethacrylic acid by oxidation of 1-butane

The terms "conversion," "selectivity," and "yield" are defined as follows.

$$\text{Conversion (mol\%)} = (\text{Number of mols of butane consumed by reaction/number of mols of butane fed to the reaction}) \times 100$$

$$\text{Selectivity of methacrolein (mol\%)} = (\text{Number of mols of formed methacrolein/number of mols of butane consumed by reaction}) \times 100$$

$$\text{Selectivity of methacrylic acid (mol\%)} = (\text{Number of mols of formed methacrylic acid/number of mols of butane consumed by reaction}) \times 100$$

$$\text{Yield (mol\%)} = (\text{Conversion} \times \text{Selectivity}) \times 100$$

A sample, 10 g in weight, of each metal ion-exchanged vanadium-phosphorus oxides obtained in Example 1 and Examples 4 - 6 was molded in the form of pellets, 5 mm in diameter and 5 mm in height. A flow type reaction vessel was packed with the pellets. A mixed gas comprising 1-butane and steam with air having a 1-butane concentration of 20 vol% and a steam concentration of 10 vol% was introduced at a spatial velocity of 2000 hr$^{-1}$ into the reaction vessel to induce gaseous-phase oxidation of n-butane. The results are shown in Table 5.

Table 5

| Example | Catalyst | Reaction temperature (°C) | Conversion (%) | Selectivity(%) | |
|---|---|---|---|---|---|
| | | | | Methacrolein | Methacrylic acid |
| Example 11 | Catalyst of Ex.1 (Co) | 450 | 15 | 11 | 3.5 |
| Example 12 | Catalyst of Ex.4 (Ni) | 450 | 10 | 14 | 2.5 |
| Example 13 | Catalyst of Ex.5 (Cu) | 450 | 17 | 19 | 4.0 |
| Example 14 | Catalyst of Ex.6 (Zn) | 450 | 19 | 10 | 1.5 |

The entire disclose of Japanese Patent Application Nos. 8-52001 and 8-52002 both filed on March 8, 1996 including specification, claims, drawing and summary are incorporated herein by reference in its entirety.

**Claims**

1.  A metal ion-exchanged phosphorus-vanadium compound obtained by treating vanadyl hydrogen orthophosphate hydrate represented by the formula (1)

$$VOHPO_4 \cdot nH_2O \qquad (1)$$

wherein n fulfills the expression, $0 \leqq n \leqq 2.0$, with an aqueous divalent metal salt solution thereby effecting the exchange of $H^+$ present between the layers of said vanadyl hydrogen orthophosphate hydrate and drying the resultant ion exchanger.

2.  A compound according to claim 1, wherein the interlayer distance is in the range of 7.0 to 8.2 Å.

3.  A metal ion-exchanged phosphorus-vanadium compound obtained by treating vanadyl hydrogen orthophosphate hydrate represented by the formula (1)

$$VOHPO_4 \cdot nH_2O \qquad (1)$$

wherein n fulfills the expression, $0 \leqq n \leqq 2.0$, with an aqueous divalent metal salt solution thereby effecting the exchange of $H^+$ present between the layers of said vanadyl hydrogen orthophosphate hydrate, drying the resultant ion exchanger, and calcining the dried ion exchanger.

4.  A compound according to claim 1 or claim 3, wherein said divalent metal is at least one member selected from the group consisting of cobalt, nickel, copper, zinc, manganese, iron, magnesium, palladium, platinum, and germanium.

5.  A method according to claim 1 or claim 3, wherein said drying is carried out at a temperature in the range of 150° to 250 °C.

6.  A method according to claim 3, wherein said calcination is carried out in the presence of a hydrocarbon under an reducing atmosphere at a temperature in the range of 350° to 600 °C.

7.  A solid catalyst formed of a metal ion-exchanged phosphorus-vanadium compound set forth in any of claims 1 - 6.

8.  A catalyst for the gaseous-phase partial oxidation of a hydrocarbon, formed of a metal ion-exchanged phosphorus-vanadium compound set forth in any of claims 1 - 6.

**Patentansprüche**

1.  Durch Metallionenaustausch gewonnene Phosphor-Vanadiumverbindung, erhalten durch Behandlung von Vanadylhydrogenorthophosphathydrat der Formel (1)

$$VOHPO_4.nH_2O \qquad (1)$$

worin n den Ausdruck $0 \leq n \leq 2,0$ erfüllt, mit einer wässerigen Lösung eines zweiwertigen Metalls, wodurch der Austausch der zwischen den Schichten des Vanadylhydrogenorthophosphathydrats vorliegenden Ionen $H^+$ bewirkt wird, und Trocknen des erhaltenen Ionenaustauschers.

2.  Verbindung gemäß Anspruch 1, bei der der Abstand zwischen den Schichten im Bereich von 7,0 bis 8,2 Å liegt.

**3.** Durch Metallionenaustausch gewonnene Phosphor-Vanadiumverbindung, erhalten durch Behandlung von Vanadylhydrogenorthophosphat-hydrat der Formel (1)

$$VOHPO_4.nH_2O \qquad (1)$$

worin n den Ausdruck $0 \leq n \leq 2,0$ erfüllt, mit einer wässerigen Lösung eines zweiwertigen Metalls, wodurch der zwischen den Schichten des Vanadylhydrogenorthophosphathydrats vorliegenden Ionen $H^+$ bewirkt wird, Trock-nen des erhaltenen Ionenaustauschers und Kalzinieren des getrockneten Ionenaustauschers.

**4.** Verbindung des Anspruchs 1 oder 3, bei der das zweiwertige Metall mindestens ein aus der Gruppe ausgewähltes Metall ist, die aus Kobalt, Nickel, Kupfer, Zink, Mangan, Eisen, Magnesium, Palladium, Platin und Germanium besteht.

**5.** Verfahren gemäß Anspruch 1 oder 3, bei dem die Trocknung bei einer Temperatur im Bereich von $150°$ bis $250°C$ durchgeführt wird.

**6.** Verfahren gemäß Anspruch 3, bei dem die Kalzinierung in Gegenwart eines Kohlenwasserstoffe unter einer reduzierenden Atmosphäre bei einer Temperatur im Bereich von $350°C$ bis $600°C$ durchgeführt wird.

**7.** Fester Katalysator, gebildet aus einer in einem der Ansprüche 1 bis 6 dargelegten, durch Metallionenaustausch gewonnenen Phosphor-Vanadiumverbindung.

**8.** Katalysator zur partiellen Gasphasenoxidation eines Kohlenwasserstoffs, gebildet aus einer der in einem der Ansprüche 1 bis 6 dargelegten, durch Metallionenaustausch gewonnenen Phosphor-Vanadiumverbindung.

**Revendications**

**1.** Composé de phosphore et de vanadium contenant un ion métallique déposé par échange d'ions obtenu en traitant l'hydrate d'hydrogénoorthophosphate de vanadyle représenté par la formule (I)

$$VOHPO_4-nH_2O \qquad (I)$$

dans laquelle n répond à l'expression, $0 \leq n \leq 2,0$, avec une solution aqueuse de sel métallique divalent, en effectuant de cette façon l'échange de $H^+$ présent entre les couches dudit hydrate d'hydrogénoorthophosphate de vanadyle et en séchant l'échangeur d'ions résultant.

**2.** Composé selon la revendication 1, dans lequel la distance entre couches est dans l'intervalle de 7,0 à 8,2 Angström.

**3.** Composé de phosphore et de vanadium contenant un ion métallique déposé par échange d'ions obtenu en traitant l'hydrate d'hydrogénoorthophosphate de vanadyle représenté par la formule (I)

$$VOHPO_4 \cdot nH_2O \qquad (I)$$

dans laquelle n répond à l'expression, $0 \leq n \leq 2,0$, avec une solution aqueuse de sel métallique divalent, en effectuant de cette façon l'échange de $H^+$ présent entre les couches dudit hydrate d'hydrogénoorthophosphate de vanadyle, en séchant l'échangeur d'ions résultant et en calcinant l'échangeur d'ions séché.

**4.** Composé selon la revendication 1 ou la revendication 3, dans lequel ledit métal divalent est au moins un membre choisi parmi le groupe consistant en cobalt, nickel, cuivre, zinc, manganèse, fer, magnésium, palladium, platine et germanium.

**5.** Procédé selon la revendication 1 ou la revendication 3, dans lequel ledit séchage est effectué à une température dans l'intervalle de $150°C$ à $250°C$.

**6.** Procédé selon la revendication 3, dans lequel ladite calcination est effectuée en présence d'un hydrocarbure sous une atmosphère réductrice à une température dans l'intervalle de 350°C à 600°C.

**7.** Catalyseur solide formé d'un composé de phosphore et de vanadium contenant un ion métallique déposé par échange d'ions exposé dans l'une quelconque des revendications 1-6.

**8.** Catalyseur pour l'oxydation partielle en phase gazeuse d'un hydrocarbure, formé d'un composé de phosphore et de vanadium contenant un ion métallique déposé par échange d'ions exposé dans l'une quelconque des revendications 1-6.

# FIG.1

VOHPO$_4$ • 0.5H$_2$O

◯V  • P  ◌ O  ◕ OH  ⊙H$_2$O  ● M$^{2+}$

# FIG.2

5.70Å

VOHPO$_4$ • 0.5H$_2$O

◯V  • P  ◌ O  ◕ OH  ⊙H$_2$O  ● M$^{2+}$

# FIG.3

VOHPO$_4$ • 1.5H$_2$O

+M$^{2+}$

VOM$^{2+}_{1/2}$PO$_4$ • 1.5H$_2$O

○ V  • P  ○ O  ● OH  ⊙ H$_2$O  ● M$^{2+}$

# FIG.4

EP 0 794 151 B1

# FIG.5

EP 0 794 151 B1